# EUROPÄISCHE PATENTSCHRIFT

(11) **EP 4 076 674 B1**
(45) Veröffentlichungstag und Bekanntmachung des Hinweises auf die Patenterteilung: **03.12.2025**
(21) Anmeldenummer: 20792647.8
(22) Anmeldetag: 16.10.2020
(51) Int. Cl.: A61Q 5/06, A61K 8/73, A61K 8/58, A61K 8/34, A61K 8/81, A61K 8/19, A61K 8/04

(54) **PIGMENTSUSPENSION UND EIN VERFAHREN ZUM FÄRBEN VON KERATINISCHEM MATERIAL UNTER EINSATZ DER PIGMENTSUSPENSION**
PIGMENT SUSPENSION AND A METHOD FOR COLORING KERATINOUS MATERIAL USING THE PIGMENT SUSPENSION
SUSPENSION DE PIGMENT ET PROCÉDÉ DE COLORATION DE LA MATIÈRE KÉRATINIQUE À L'AIDE DE LA SUSPENSION DE PIGMENT

(30) Priorität: 16.12.2019 DE 102019219712
(43) Veröffentlichungstag der Anmeldung: 26.10.2022
(73) Patentinhaber: Henkel AG & Co. KGaA, 40589 Düsseldorf (DE)
(72) Erfinder: KROHN, Rene, 22848 Norderstedt (DE); HIPPE, Thomas, 25482 Appen (DE); BRENDER, Jessica, 22763 Hamburg (DE); HOEPFNER, Stefan, 22523 Hamburg (DE)
(86) Internationale Anmeldenummer: PCT/EP2020/079172
(87) Internationale Veröffentlichungsnummer: WO 2021/121720

(56) Entgegenhaltungen:
- WO-A1-2016/133812
- DE-A1- 102018 207 025

## Beschreibung

Gegenstand der vorliegenden Anmeldung ist eine Pigmentsuspension, umfassend ein Pigment und ein Trägermedium. Ein weiterer Gegenstand ist ein Verfahren zum Färben von keratinischem Material unter Einsatz der Pigmentsuspension.

Pigmente, insbesondere Metall-haltige Pigmente, werden häufig in Lacken, Farben, Druckfarben, Pulverlacken, Kosmetika oder Kunststoffen zur Farbgebung eingesetzt. Bei Farben, Lacken, Druckfarben, Kosmetika und Pulverlacken handelt es sich um flüssige oder pulverförmige Beschichtungsstoffe, die auf Oberflächen aufgebracht werden, um sowohl verbesserte oder veränderte optische als auch physikalische Eigenschaften zu erhalten.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit Tensid-haltigen Reinigungsmitteln wieder rückstandslos entfernen. Unter dem Namen Haar-Mascara sind verschiedene Produkte dieses Typs auf dem Markt erhältlich.

Metallische Glanzpigmente oder Metalleffektpigmente finden breite Anwendung in vielen Bereichen der Technik. Sie werden beispielsweise zur Einfärbung von Lacken, Druckfarben, Tinten, Kunststoffen, Gläsern keramischen Produkten und Zubereitungen der dekorativen Kosmetik wie Nagellack eingesetzt.

Sie zeichnen sich vor allem durch ihren reizvollen winkelabhängigen Farbeindruck (Goniochromie) und ihren metallartig wirkenden Glanz aus.

Haare mit einem Metallic Finish oder metallischen Reflexen sind im Trend. Durch den Metallic-Ton wirkt das Haar dicker und glänzender.

Für Anwendungen im kosmetischen Bereich, beispielsweise bei der Farbveränderung von keratinischen Fasern mit Pigmenten, ist es wichtig, dass die Pigmente dem Anwender in einer lagerstabilen und dosierfähigen Form bereitgestellt werden. Dies kann insbesondere in Form einer lagerstabilen Pigmentsuspension erfolgen.

Für die Herstellung anorganischer Pigmentsuspensionen werden üblicherweise gemahlene Pigmentpulver und Wasser verwendet. Gegebenenfalls müssen organische oder anorganische Dispergierhilfsmittel in kleinen Mengen zugesetzt werden.

Aufgabe der vorliegenden Erfindung ist es, Pigmentsuspensionen bereitzustellen, die sich einfach und kostengünstig herstellen und lagerstabil sind. Insbesondere sollten die Pigmente nicht agglomerieren und/oder sich absetzen. Das Trägermedium sowie eventuell vorhandene Hilfsmittel sollten kompatibel mit den Anwendungsmodalitäten der Pigmentsuspension sein.

Es hat sich gezeigt, dass Pigmentsuspensionen, die einen C1-C10-Alkohol, ein Diol und Wasser als Trägermedium sowie ein Verdickungsmittel enthalten, diesen Anforderungen gerecht werden.

Entsprechend ist ein erster Gegenstand der Anmeldung eine umfassend
a) mindestens eine farbgebende Verbindung aus der Gruppe der Pigmente, wobei das Pigment ein Substratplättchen aus natürlichem Glimmer oder synthetischem Glimmer umfasst,
b) mindestens einen C₁-C₁₀-Alkohol,
c) mindestens ein Diol, ausgewählt aus der Gruppe bestehend aus Ethylenglycol, 1,2-Propylenglycol, 1,3-Propylenglycol und Mischungen daraus,
d) mindestens ein Verdickungsmittel und
e) Wasser.

Als ersten erfindungswesentlichen Inhaltsstoff enthalten die Pigmentsuspensionen mindestens eine ausgewählte farbgebende Verbindung aus der Gruppe der Pigmente.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Das mindestens eine Pigment weist ein Substratplättchen auf.

Das Substratplättchen kann prinzipiell aus jedem Material, das in Plättchenform gebracht werden kann, aufgebaut sein.

Sie können natürlichen Ursprungs, aber auch synthetisch hergestellt sein. Die Substratplättchen sind aus einem Glimmer aufgebaut. Der Glimmer kann natürlichem oder synthetischen Ursprungs sein.

Das Pigment weist ein Substratplättchen auf, wobei das Substratplättchen natürlichen Glimmer oder synthetischen Glimmer umfasst. I

In einer besonders bevorzugten Ausführungsform weist das Pigment ein Substratplättchen aus synthetischem Glimmer (INCI: Synthetic Fluorphlogopite) auf.

Das Substratplättchen aus Glimmer weist vorzugsweise eine durchschnittliche Dicke von 50 bis 1500 nm und mehr bevorzugt von 90 bis 1000 nm auf.

Die Größe des Substratplättchens kann auf den jeweiligen Anwendungszweck, beispielsweise dem gewünschten Effekt auf einem keratinischen Material, abgestimmt werden. In der Regel haben die Substratplättchen aus einem Glimmer einen mittleren größten Durchmesser von etwa 1 bis 200 µm, insbesondere etwa 5 bis 100 µm und noch mehr bevorzugt von etwa 5 bis 25 µm.

In einer bevorzugten Ausführungsform beträgt der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Die Substratplättchen können verschiedene Formen aufweisen.

Durch eine Beschichtung können die Oberflächeneigenschaften und/oder optischen Eigenschaften des Pigments verändert sowie die mechanische und chemische Belastbarkeit der Pigmente erhöht werden. Es können beispielsweise lediglich die obere und/oder untere Seite des Substratplättchens beschichtet sein, wobei die Seitenflächen ausgespart sind. Vorzugsweise ist die gesamte Oberfläche der gegebenenfalls passivierten Substratplättchen, einschließlich der Seitenflächen, von der Schicht bedeckt. Die Substratplättchen sind vorzugsweise vollständig von der Beschichtung umhüllt.

Die Beschichtung kann aus einer oder aus mehreren Schichten bestehen. In einer bevorzugten Ausführungsform weist die Beschichtung lediglich eine Schicht A auf. In einer ebenfalls bevorzugten Ausführungsform weist die Beschichtung insgesamt mindestens zwei, vorzugsweise zwei oder drei, Schichten auf. Es kann bevorzugt sein, die Beschichtung zwei Schichten A und B aufweist, wobei die Schicht B von der Schicht A verschieden ist. Vorzugsweise befindet sich Schicht A zwischen der Schicht B und der Oberfläche des Substratplättchens. In noch einer bevorzugten Ausführungsform weist die Beschichtung drei Schichten A, B und C auf. In dieser Ausführungsform befindet sich zwischen der Schicht B und der Oberfläche des Substratplättchens die Schicht A und auf der Schicht B befindet sich eine Schicht C, die von der darunterliegenden Schicht B verschieden ist.

Als Materialien für die Schichten A und gegebenenfalls B und C eignen sich alle Substanzen, die dauerhaft auf die Substratplättchen aufgebracht werden können. Die Materialien sollten mit Vorzug filmartig aufbringbar sein. Vorzugsweise ist die gesamte Oberfläche der gegebenenfalls passivierten Substratplättchen, einschließlich der Seitenflächen, von der Schicht A oder von den Schichten A und B oder von den Schichten A, B und C umhüllt.

Die Schichten können insbesondere jeweils mindestens ein Metalloxid(hydrat) enthalten.

Es ist bevorzugt, dass das Metalloxid(hydrat) ausgewählt ist aus der Gruppe bestehend aus Silicium(di)oxid, Siliciumoxidhydrat, Aluminiumoxid, Aluminiumoxidhydrat, Boroxid, Germaniumoxid, Manganoxid, Magnesiumoxid, Eisenoxid, Cobaltoxid, Chromoxid, Titandioxid, Vanadiumoxid, Zirkonoxid, Zinnoxid, Zinkoxid und deren Gemischen.

Im Fall von Pigmenten mit einem Substratplättchen aus Glimmer, insbesondere synthetischem Glimmer, umfasst die Schicht A ein Metalloxid(hydrat) ausgewählt aus der Gruppe bestehend aus Titandioxid (TiO₂), Eisenoxid (Fe₂O₃ und/oder Fe₃O₄) und Mischungen daraus. In einer ganz bevorzugten Ausführungsform umfasst Schicht A Titandioxid (TiO₂) und/oder Eisenoxid (Fe₂O₃). In einer äußerst bevorzugten Ausführungsform umfasst Schicht A Titandioxid (TiO₂).

Schicht B, falls vorhanden, ist auch im Fall von Pigmenten mit einem Substratplättchen aus Glimmer von der ersten Metalloxid(hydrat)schicht verschieden.

Für Schicht B geeignete Metalloxid(hydrat)e sind Zinnoxid (SnO₂), Siliciumoxid (SiO₂), Aluminiumoxid (Al₂O₃) und/oder Eisenoxid (Fe₂O₃ und/oder Fe₃O₄). Entsprechend ist es bevorzugt, dass Schicht B ein Metalloxid(hydrat) ausgewählt aus der Gruppe bestehend aus Zinnoxid (SnO₂), Siliciumoxid (SiO₂), Aluminiumoxid (Al₂O₃), Eisenoxid (Fe₂O₃ und/oder Fe₃O₄) und Mischungen daraus enthält. Es ist insbesondere bevorzugt, dass Schicht B im Fall von Pigmenten mit einem Substratplättchen aus Glimmer, vorzugsweise synthetischem Glimmer, Zinnoxid (SnO₂) enthält.

Schicht B kann ferner einen selektiv absorbierenden Farbstoff oder ein selektiv absorbierendes Pigment enthalten. Geeignete Farbstoffe und/oder Pigmente umfassen beispielsweise Karmin, Eisen(III)hexacyanidoferrat(II/III) und Chromoxidgrün (Cr₂O₃).

Die Pigmente mit einem Substratplättchen aus Glimmer können eine weitere Schicht C aufweisen, die als Schutzschicht fungiert und ein Metalloxid(hydrat) oder ein Polymer, beispielsweise einen Kunstharz, umfasst. Geeignete Metalloxid(hydrate)e sind beispielsweise Silicium(di)oxid, Siliciumoxidhydrat, Aluminiumoxid, Aluminiumoxidhydrat, Zinkoxid, Zinnoxid, Titandioxid, Zirkonoxid, Eisen(III)oxid und Chrom(III)oxid. Bevorzugt ist Siliciumdioxid.

Es ist insbesondere bevorzugt, dass ein Pigment mit einem Substratplättchen aus synthetischem Glimmer (INCI: Synthetic Fluorphlogopite) eine Schicht A, umfassend Titandioxid (TiO₂) aufweist.

Es ist ebenfalls bevorzugt, dass ein Pigment mit einem Substratplättchen aus synthetischem Glimmer (INCI: Synthetic Fluorphlogopite) eine Schicht A, umfassend Eisen(III)oxid (Fe₂O₃) aufweist.

Es ist auch bevorzugt, dass ein Pigment mit einem Substratplättchen aus synthetischem Glimmer (INCI: Synthetic Fluorphlogopite) eine Schicht A, umfassend Titandioxid (TiO₂) und Eisen(III)oxid (Fe₂O₃), und eine Schicht B, umfassend Zinndioxid (SnO₂), aufweist.

Es ist äußerst bevorzugt, dass ein Pigment mit einem Substratplättchen aus synthetischem Glimmer (INCI: Synthetic Fluorphlogopite) eine Schicht A, umfassend Titandioxid (TiO₂), und eine Schicht B, umfassend Zinndioxid (SnO₂), aufweist.

Bevorzugt geeignete Pigment mit einem Substratplättchen aus synthetischem Glimmer sind beispielsweise Timiron^{®} SynWhite Satin, Colorona^{®} SynCopper und/oder Colorona^{®} SynBronze von Merck.

Die Menge an Pigment in der Pigmentsuspension hängt insbesondere von der Art des Pigments/der Pigmente und dessen/deren Anwendungszweck ab. Vorzugsweise liegt die Menge an Pigment zwischen 1 und 90 Gew.-%, mehr bevorzugt zwischen 5 und 80 Gew-% und ganz besonders bevorzugt zwischen 10 und 70 Gew.-%, jeweils bezogen auf das Gesamtgewicht der Pigmentsuspension.

Neben den genannten, Pigmenten mit Substratplättchen aus Glimmer, können weitere farbgebende Verbindungen in der Pigmentsuspension enthalten sein. Die weiteren farbgebenden Verbindungen können beispielsweise weitere anorganische Pigmente, organische Pigmente und/oder direktziehende Farbstoffe umfassen.

Als zweiten erfindungswesentlichen Inhaltsstoff umfasst die Pigmentsuspension mindestens einen C₁-C₁₀-Alkohol.

Der C₁-C₁₀ Alkohol ist vorzugsweise ein aliphatischer C₁-C₁₀-Alkohol, der linear oder verzweigt sowie gesättigt oder ungesättigt sein kann.

Bevorzugte C₁-C₁₀ Alkohole sind ausgewählt aus der Gruppe bestehend aus Methanol, Ethanol, 1-Propanol, 2-Propanol, 1-Butanol, 2-Butanol, 2-Methylpropan-1-ol, 2-Methylpropan-1-ol (tert.-Butanol), 1-Pentanol, 2-Pentanol, 3-Pentanol, 3-Methylbutan-1-ol, 2-Methylbutan-1-ol, 2,2-Dimethylpropan-1-ol, 3-Methylbutan-2-ol, 2-Methylbutan-2-ol, 1-Hexanol, 2-Hexanol, 3-Hexanol, 2-Methylpentan-1-ol, 3-Methylpentan-1-ol, 4-Methylpentan-1-ol, 2-Methylpentan-2-ol, 3-Methylpentan-2-ol, 4-Methylpentan-2-ol, 2-Methylpentan-3-ol, 3-Methylpentan-3-ol, 2,2-Dimethylbutan-1-ol, 2,3-Dimethylbutan-1-ol, 3,3-Dimethylbutan-1-ol, 2,3-Dimethylbutan-2-ol, 3,3-Dimethylbutan-2-ol, 2-Ethylbutan-1-ol, 1-Heptanol, 2-Heptanol, 3-Heptanol, 4-Heptanol, 1-Octanol, 2-Octanol, 1-Nonanol, 1-Decanol, 2-Methylhexan-2-ol, 2-Methylheptan-2-ol, 3-Methyl-3-pentanol und Mischungen davon.

Von den C₁-C₁₀ Alkoholen enthält die Pigmentsuspension vorzugsweise mindestens einen C₁-C₁₀ Alkohol ausgewählt aus der Gruppe bestehend Methanol, Ethanol, 1-Propanol, 2-Propanol, 1-Butanol, 2-Butanol, 2-Methylpropan-1-ol, 2-Methylpropan-1-ol (tert.-Butanol) und Mischungen davon.

In einer äußerst bevorzugten Ausführungsform ist die Pigmentsuspension dadurch gekennzeichnet, dass die Pigmentsuspension mindestens einen C₁-C₁₀-Alkohol ausgewählt aus der Gruppe bestehend aus Ethanol, 2-Propanol und Mischungen daraus enthält.

Besonders stabile Pigmentsuspensionen konnten erhalten werden, wenn die Pigmentsuspension - bezogen auf das Gesamtgewicht der Pigmentsuspension - ein oder mehrere C₁-C₁₀-Alkohole in einer Gesamtmenge von 1 bis 80 Gew.-%, bevorzugt von 5 bis 60 Gew.-% und ganz besonders bevorzugt von 10 bis 40 Gew.-% enthält.

Als dritten erfindungswesentlichen Inhaltsstoff umfasst die Pigmentsuspension mindestens ein ausgewähltes Diol.

Ein Diol ist eine chemische Verbindung mit zwei Hydroxylgruppen (-OH-Gruppen). Ein aliphatisches Diol wird auch als Glykol bezeichnet.

Die Pigmentsuspension ist dadurch gekennzeichnet, dass die Pigmentsuspension mindestens ein Diol ausgewählt aus der Gruppe bestehend aus Ethylenglycol, 1,2-Propylenglycol und 1,3-Propylenglycol enthält.

Besonders stabile Pigmentsuspensionen konnten erhalten werden, wenn die Pigmentsuspension - bezogen auf das Gesamtgewicht der Pigmentsuspension -ein oder mehrere Diole in einer Gesamtmenge von 1 bis 50 Gew.-%, bevorzugt von 1 bis 40 Gew.-%, weiter bevorzugt von 1 bis 30 Gew.-% und ganz besonders bevorzugt von 1 bis 20 Gew.-% enthält.

Als vierten erfindungswesentlichen Inhaltsstoff umfasst die Pigmentsuspension mindestens ein Verdickungsmittel.

Geeignete Verdickungsmittel umfassen insbesondere chemisch modifizierte Cellulosen, wie beispielsweise Propylcellulose, Methylethylcellulose, Carboxymethylcellulose, Hydroxyethylcellulose, Methylhydroxyethylcellulose, Ethylhydroxyethylcellulose, Hydroxypropylcellulose, Hydroxypropylmethylcellulose, Carboxymethylhydroxyethylcellulose, Sulfoethylcellulose, Carboxymethylsulfoethylcellulose, Hydroxypropylsulfoethylcellulose, Hydroxyethylsulfoethylcellulose, Methylethylhydroxyethylcellulose, Methlylsulfoethylcellulose und/oder Ethylsulfoethylcellulose.

In einer bevorzugten Ausführungsform ist eine Pigmentsuspension dadurch gekennzeichnet, dass die Pigmentsuspension ein Verdickungsmittel ausgewählt aus der Gruppe bestehend aus Propylcellulose, Methylethylcellulose, Carboxymethylcellulose, Hydroxyethylcellulose, Methylhydroxyethylcellulose, Ethylhydroxyethylcellulose, Hydroxypropylcellulose, Hydroxypropylmethylcellulose, Carboxymethylhydroxyethylcellulose, Sulfoethylcellulose, Carboxymethylsulfoethylcellulose, Hydroxypropylsulfoethylcellulose, Hydroxyethylsulfoethylcellulose, Methylethylhydroxyethylcellulose, Methlylsulfoethylcellulose, Ethylsulfoethylcellulose und Mischungen daraus enthält.

Besonders geeignete Verdickungsmittel sind ausgewählt aus Hydroxyethylcellulose, Hydroxypropylcellulose, Hydroxypropylmethylcellulose und Mischungen daraus.

In einer besonders bevorzugten Ausführungsform ist eine Pigmentsuspension dadurch gekennzeichnet, dass das Mittel (a) und/oder das Mittel (b) ferner ein Verdickungsmittel ausgewählt aus der Gruppe bestehend aus Hydroxyethylcellulose, Hydroxypropylcellulose, Hydroxypropylmethylcellulose und Mischungen daraus enthält.

Die Menge an Verdickungsmittel liegt bevorzugt zwischen 0,1 und 10 Gew.-% und mehr bevorzugt zwischen 0,25 und 5 Gew.-% jeweils bezogen auf die Gesamtmenge an Pigmentsuspension.

Als fünften erfindungswesentlichen Inhaltsstoff umfasst die Pigmentsuspension Wasser. Die Menge an Wasser beträgt bevorzugt von 1 bis 50 Gew.-%, bevorzugt von 1 bis 40 Gew.-%, weiter bevorzugt von 1 bis 30 Gew.-% und ganz besonders bevorzugt von 1 bis 20 Gew.-%, jeweils bezogen auf die Gesamtmenge an Pigmentsuspension.

Es hat sich gezeigt, dass die Pigmente, mit Substratplättchen natürlichem Glimmer oder synthetischem Glimmer mit Hilfe der Pigmentsuspension stabil gelagert und genau dosiert werden können.

Ein zweiter Gegenstand der vorliegenden Erfindung betrifft ein Verfahren zum Färben von keratinischem Material, insbesondere menschlichen Haaren, umfassend die folgenden Schritte:
- Anwendung eines Mittels (a) auf dem keratinischem Material, wobei das Mittel (a) enthält:
   (a1) mindestens ein organisches C₁-C₆-Alkoxysilan, und
- Anwendung eines Mittels (b) auf dem keratinischen Material, wobei das Mittel (b) enthält:
   (b1) mindestens ein Versiegelungsreagenz,
wobei mindestens eines der Mittel (a) und (b) ferner eine Pigmentsuspension nach einem der Ansprüche 1 bis 4 umfasst.

Das Verfahren kann ein Mittel (a) umfassen, welches durch Kombination einer Pigmentsuspension gemäß der vorliegenden Erfindung mit einem oder mehreren organischen C₁-C₆-Alkoxysilanen (a1) und/oder deren Kondensationsprodukten hergestellt wurde.

Alternativ kann das Verfahren kann ein Mittel (b) umfassen, welches durch Kombination einer Pigmentsuspension gemäß der vorliegenden Erfindung mit einem oder mehreren Versiegelungsreagenzien (b1) hergestellt wurde.

Das in dem Verfahren eingesetzte Mittel (a) ist dadurch gekennzeichnet, dass es ein oder mehrere organische C₁-C₆-Alkoxysilane und/oder deren Kondensationsprodukte enthält.

Bei dem oder den organischen C₁-C₆-Alkoxysilanen handelt es sich um organische, nicht polymere Siliciumverbindungen, die bevorzugt aus der Gruppe der Silane mit einem, zwei oder drei Siliciumatomen ausgewählt sind

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die erfindungsgemäßen organische Siliciumverbindungen sind bevorzugt Verbindungen, die ein bis drei Siliciumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die WasserstoffAtome ganz oder teilweise durch organische Gruppen wie beispielsweise (substituierte) Alkylgruppen und/oder Alkoxygruppen ersetzt.

Kennzeichnend für die C₁-C₆-Alkoxysilane ist, dass mindestens eine C₁-C₆-Alkoxygruppe direkt an ein Siliciumatom gebunden vorliegt. Die erfindungsgemäßen C₁-C₆-Alkoxysilane umfassen damit mindestens eine Struktureinheit R'R"R‴Si-O-(C₁-C₆-Alkyl) wobei die Reste R', R" und R‴ für die drei übrigen Bindungsvalenzen des Siliciumatoms stehen.

Das oder diese an das Siliciumatom gebundenen C₁-C₆-Alkoxygruppen sind sehr reaktiv und werden in Anwesenheit von Wasser mit hoher Geschwindigkeit hydrolysiert, wobei die Reaktionsgeschwindigkeit unter anderem auch von der Anzahl der hydrolysierbaren Gruppen pro Molekül abhängt. Handelt es sich bei der hydrolysierbaren C₁-C₆-Alkoxy-Gruppe um eine Ethoxygruppe, so enthält die organische Siliciumverbindung bevorzugt eine Struktureinheit R'R"R‴Si-O-CH2-CH3. Die Reste R', R" und R‴ stellen wieder die drei übrigen freien Valenzen des Siliciumatoms dar.

Bereits der Zusatz geringer Wassermengen führt zunächst zur Hydrolyse und dann zu einer Kondensationsreaktion der organischen Alkoxysilane untereinander. Aus diesem Grund können sowohl die organischen Alkoxysilane als auch deren Kondensationsprodukte in dem Mittel enthalten sein.

Unter einem Kondensationsprodukt wird ein Produkt verstanden, dass durch Reaktion von mindestens zwei organischen C₁-C₆-Alkoxysilanen unter Abspaltung von Wasser und/oder unter Abspaltung von einem C₁-C₆-Alkanol entsteht.

Die Kondensationsprodukte können beispielsweise Dimere, aber auch Trimere oder Oligomere sein, wobei die Kondensationsprodukte mit den Monomeren im Gleichgewicht stehen.

Abhängig von der eingesetzten bzw. in der Hydrolyse verbrauchten Wassermenge verschiebt sich das Gleichgewicht von monomerem C₁-C₆-Alkoxysilan zu Kondensationsprodukt.

Im Rahmen einer ganz besonders bevorzugten Ausführungsform ist ein Verfahren dadurch gekennzeichnet, dass das Mittel (a) ein oder mehrere organische C₁-C₆-Alkoxysilane enthält, die aus Silanen mit einem, zwei oder drei Siliciumatomen ausgewählt sind, wobei die organische Siliciumverbindung außerdem eine oder mehrere basische chemische Funktionen umfasst.

Bei dieser basischen Gruppe kann es sich beispielsweise um eine Aminogruppe, eine Alkylaminogruppe oder um eine Dialkylaminogruppe handeln, die bevorzugt über einen Linker mit einem Siliciumatom verbunden ist. Bevorzugt handelt es sich bei der basischen Gruppe um eine Aminogruppe, eine C₁-C₆-Alkylaminogruppe oder um eine Di(C₁-C₆)alkylaminogruppe.

Ein ganz besonders bevorzugtes Mittel (a), welches in dem Verfahren eingesetzt werden kann, ist dadurch gekennzeichnet, dass das kosmetische Mittel ein oder mehrere organische C₁-C₆-Alkoxysilane enthält, die ausgewählt sind aus der Gruppe der Silane mit einem, zwei oder drei Siliciumatomen, und wobei die C₁-C₆-Alkoxysilane weiterhin eine oder mehrere basische chemische Funktionen umfassen.

Ganz besonders gute Ergebnisse konnten erhalten werden, wenn in dem Mittel (a) C₁-C₆-Alkoxysilane der Formel (S-I) und/oder (S-II) und/oder (S-IV) eingesetzt wurden. Da wie bereits zuvor beschrieben bereits bei Spuren von Feuchtigkeit eine Hydrolyse/Kondensation einsetzt, sind auch die Kondensationsprodukte der C₁-C₆-Alkoxysilane der Formel (S-I) und/oder (S-II) und/oder (S-IV) von dieser Ausführungsform mit umfasst.

In einer weiteren ganz besonders bevorzugten Ausführungsform ist ein Verfahren dadurch gekennzeichnet, dass das Mittel (a) ein oder mehrerer organische C₁-C₆-Alkoxysilane der Formel (S-I) und/oder (S-II) enthält,

R₁R₂N-L-Si(OR₃)ₐ(R₄)_{b} (S-I)

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃, R₄ unabhängig voneinander für eine C₁-C₆-Alkylgruppe stehen,
- a, für eine ganze Zahl von 1 bis 3 steht, und
- b für die ganze Zahl 3 - a steht, und
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (S-II),

wobei
- R5, R5', R5", R6, R6' und R6" unabhängig voneinander für eine C₁-C₆-Alkylgruppe stehen,
- A, A', A", A‴ und A"" unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (S-III) stehen,

   - (Aʺʺ)-Si(R₆")_{d}"(OR₅")_{c}" (S-III),

   - c, für eine ganze Zahl von 1 bis 3 steht,
   - d für die ganze Zahl 3 - c steht,
   - c' für eine ganze Zahl von 1 bis 3 steht,
   - d' für die ganze Zahl 3 - c' steht,
   - c" für eine ganze Zahl von 1 bis 3 steht,
   - d" für die ganze Zahl 3 - c" steht,
   - e für 0 oder 1 steht,
   - f für 0 oder 1 steht,
   - g für 0 oder 1 steht,
   - h für 0 oder 1 steht,
   - mit der Maßgabe, dass mindestens einer der Reste aus e, f, g und h von 0 verschieden ist, und/oder deren Kondensationsprodukte.

Die Substituenten R₁, R₂, R₃, R₄, R₅, R₅', R₅", R₆, R₆', R₆", R₇, R₈, L, A, A', A", A‴ und A"" in den Verbindungen der Formel (S-I) und (S-II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen der Formel (S-I)

R₁R₂N-L-Si(OR₃)ₐ(R₄)_{b} (S-I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L- der für eine lineare oder verzweigte, zweiwertige C₁-C₂₀-Alkylengruppe steht. Die zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung -L- zwei Bindungen eingehen kann.

Bevorzugt steht -L- für eine lineare, zweiwertige C₁-C₂₀-Alkylengruppe. Weiter bevorzugt steht -L- für eine lineare zweiwertige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die Alkoxysilane der Formel (S-I)

R₁R₂N-L-Si(OR₃)ₐ(R₄)_{b} (S-I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht die Reste R3 und R4 unabhängig voneinander für eine C₁-C₆-Alkylgruppe, Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Mittel (a) mit besonders guten Färbeeigenschaften bei keratinischen Materialien konnten hergestellt werden, wenn das Mittel (a) mindestens ein organisches C₁-C₆-Alkoxysilan der Formel (S-I) enthält, bei welchem die Reste R₃, R₄ unabhängig voneinander für eine Methylgruppe oder für eine Ethylgruppe stehen.

Weiterhin konnten Färbungen mit den besten Waschechtheiten erhalten werden, wenn das Mittel (a) mindestens ein organisches C₁-C₆-Alkoxysilan der Formel (S-I) enthält, bei welchem der Rest a für die Zahl 3 steht. In diesem Fall steht der Rest b für die Zahl 0.

In einer weiteren bevorzugten Ausführungsform ist das Mittel (a) dadurch gekennzeichnet, dass es ein oder mehrere organische C₁-C₆-Alkoxysilane der Formel (S-I) enthält,
wobei
- R₃, R₄ unabhängig voneinander für eine Methylgruppe oder für eine Ethylgruppe stehen und
- a für die Zahl 3 steht und
- b für die Zahl 0 steht.

In einer weiteren bevorzugten Ausführungsform ist ein erfindungsgemäßes Verfahren dadurch gekennzeichnet, dass das Mittel (a) mindestens ein oder mehrere organische C₁-C₆-Alkoxysilane der Formel (S-I) enthält,

R₁R₂N-L-Si(OR₃)ₐ(R₄)_{b} (S-I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für eine Ethylgruppe oder eine Methylgruppe steht,
- R₄ für eine Methylgruppe oder für eine Ethylgruppe steht,
- a für die Zahl 3 steht und
- b für die Zahl 0 steht.

Besonders gut geeignete organische Siliciumverbindungen der Formel (I) sind
- (3-Aminopropyl)triethoxysilan 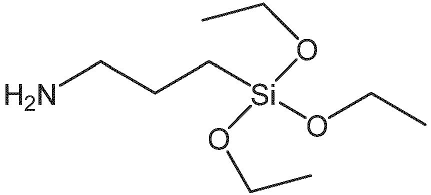
- (3-Aminopropyl)trimethoxysilan 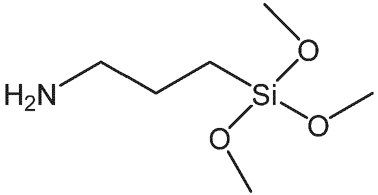
- (2-Aminoethyl)triethoxysilan 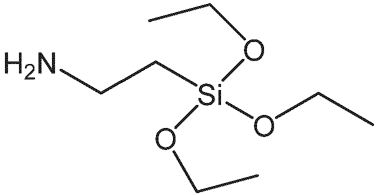
- (2-Aminoethyl)trimethoxysilan 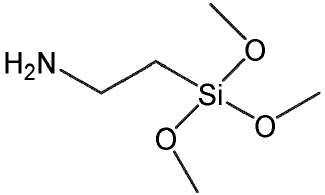
- (3-Dimethylaminopropyl)triethoxysilan 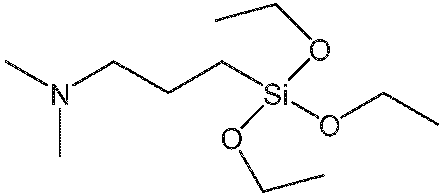
- (3-Dimethylaminopropyl)trimethoxysilan 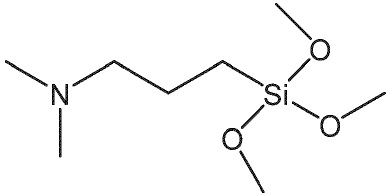
- (2-Dimethylaminoethyl)triethoxysilan. 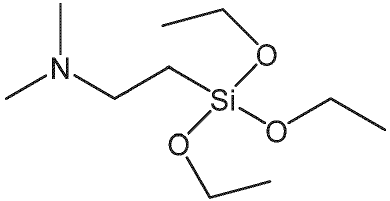
- (2-Dimethylaminoethyl)trimethoxysilan und/oder

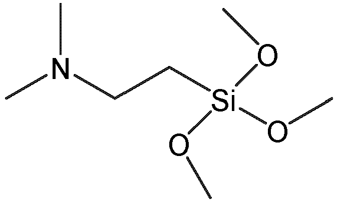

In einer weiteren bevorzugten Ausführungsform ist ein erfindungsgemäßes Verfahren dadurch gekennzeichnet, dass das Mittel (a) mindestens ein organisches C₁-C₆-Alkoxysilan der Formel (S-I) enthält, das ausgewählt ist aus der Gruppe aus
- (3-Aminopropyl)triethoxysilan
- (3-Aminopropyl)trimethoxysilan
- (2-Aminoethyl)triethoxysilan
- (2-Aminoethyl)trimethoxysilan
- (3-Dimethylaminopropyl)triethoxysilan
- (3-Dimethylaminopropyl)trimethoxysilan
- (2-Dimethylaminoethyl)triethoxysilan,
- (2-Dimethylaminoethyl)trimethoxysilan
und/oder deren Kondensationsprodukten.

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Im Rahmen einer weiteren Ausführungsform des erfindungsgemäßen Verfahrens kann Mittel (a) auch ein oder mehrere organische C₁-C₆-Alkoxysilane der Formel (S-II) enthalten,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (S-II).

Die Alkoxysilane der Formel (S-II) tragen jeweils an ihren beiden Enden die Silicium-haltigen Gruppierungen (R₅O)_{c}(R₆)_{d}Si- und -Si(R₆')_{d'}(OR₅')_{c'}.

Im Mittelteil des Moleküls der Formel (S-II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält ein bevorzugtes Alkoxysilan der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Färbungen mit den besten Waschechtheiten konnten erhalten werden, wenn die Reste c und c' beide für die Zahl 3 stehen. In diesem Fall stehen d und d' beide für die Zahl 0.

In einer weiteren bevorzugten Ausführungsform ist ein Verfahren dadurch gekennzeichnet, dass das Mittel (a) ein oder mehrere organische C₁-C₆-Alkoxysilane der Formel (S-II) enthält,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (S-II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die erfindungsgemäßen organischen Siliciumverbindung der Formel (S-IIa)

(R₅O)₃Si-(A)ₑ-[NR₇-(A')]_{f}-[O-(A")]_{g}-[NR₈-(A‴)]ₕ-Si(OR₅')₃ (S-IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erzielung von waschechten Färbeergebnissen erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die erfindungsgemäßen organischen Siliciumverbindung der Formel (S-IIb)

(R₅O)_{c}(R₆)_{d}Si-(A)-[NR₇-(A')]-Si(R₆')_{d'}(OR₅')_{c'} (S-IIb).

Die Reste A, A', A", A‴ und A"" stehen unabhängig voneinander für eine lineare oder verzweigte, zweiwertige C₁-C₂₀-Alkylengruppe. Bevorzugt stehen die Reste A, A', A", A‴ und A"" unabhängig voneinander für eine lineare, zweiwertige C₁-C₂₀-Alkylengruppe. Weiter bevorzugt stehen die Reste A, A', A", A‴ und Aʺʺ unabhängig voneinander für eine lineare zweiwertige C₁-C₆-Alkylengruppe.

Die zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung A, A', A", A‴ und Aʺʺ zwei Bindungen eingehen kann.

Besonders bevorzugt stehen die Reste A, A', A", A‴ und A"" unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und A"" für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (S-III)

- (Aʺʺ)-Si(R₆")_{d}"(OR₅")_{c}" (S-III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (S-III).

Wenn der Rest f für die Zahl 1 steht und der Rest h für die Zahl 0 steht, enthält die erfindungsgemäße organische Siliciumverbindung die Gruppierung [NR₇-(A')], aber nicht die Gruppierung -[NR₈-(A‴)]. Steht nun der Rest R7 für eine Gruppierung der Formel (III), so umfasst die organische Siliciumverbindung 3 reaktive Silan-Gruppen.

In einer weiteren bevorzugten Ausführungsform ist ein Verfahren dadurch gekennzeichnet, dass das Mittel (a) ein oder mehrere organische C₁-C₆-Alkoxysilane der Formel (S-II) enthält

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (S-III) steht.

In einer weiteren bevorzugten Ausführungsform ist ein Verfahren dadurch gekennzeichnet, dass Mittel (a) ein oder mehrere organische C₁-C₆-Alkoxysilane der Formel (S-II) enthält, wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (S-III) steht.

Gut geeignete organische Siliciumverbindungen der Formel (S-II) sind
- 3-(Trimethoxysilyl)-N-[3-(trimethoxysilyl)propyl]-1-propanamin 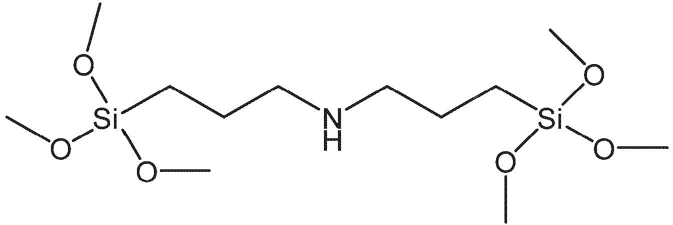
- 3-(Triethoxysilyl)-N-[3-(triethoxysilyl)propyl]-1-propanamin 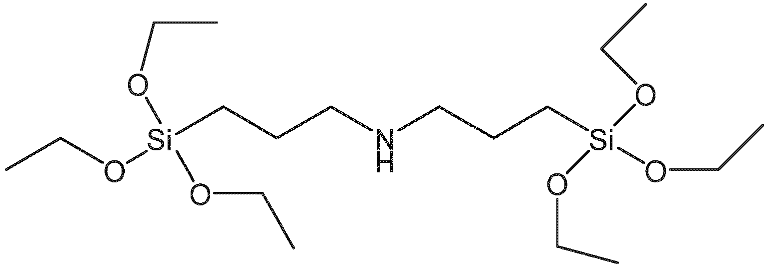
- N-Methyl-3-(trimethoxysilyl)-N-[3-(trimethoxysilyl)propyl]-1-propanamin 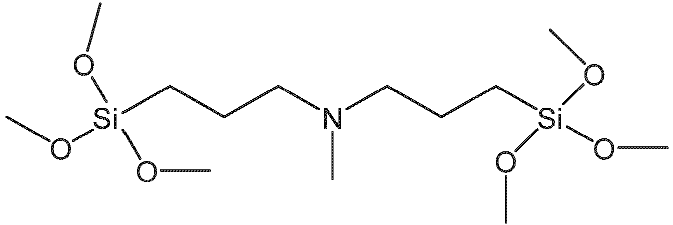
- N-Methyl-3-(triethoxysilyl)-N-[3-(triethoxysilyl)propyl]-1-propanamin 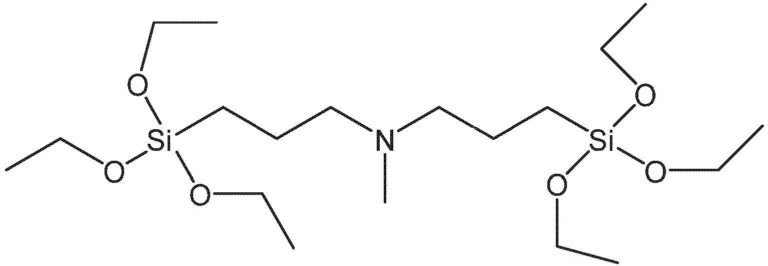
- 2-[Bis[3-(trimethoxysilyl)propyl]amino]-ethanol 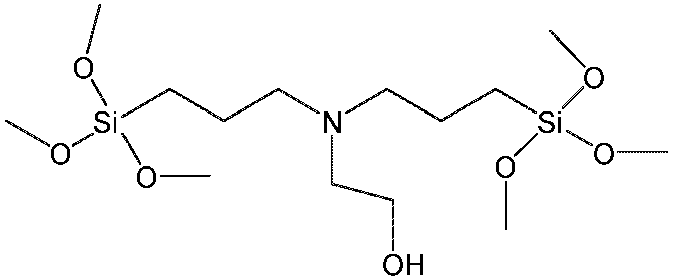
- 2-[Bis[3-(triethoxysilyl)propyl]amino]-ethanol 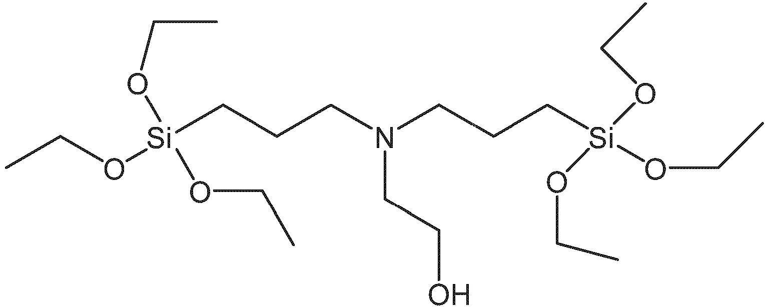
- 3-(Trimethoxysilyl)-N,N-bis[3-(trimethoxysilyl)propyl]-1-propanamin 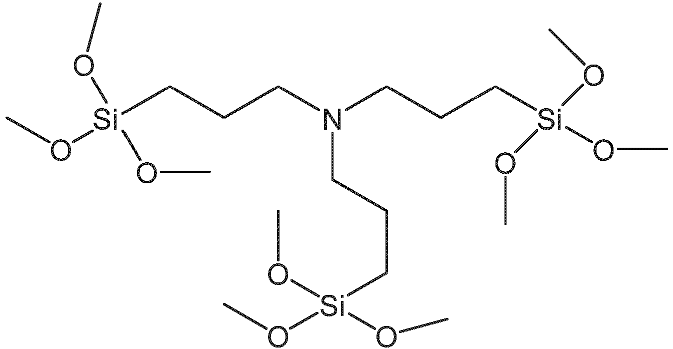
- 3-(Triethoxysilyl)-N,N-bis[3-(triethoxysilyl)propyl]-1 -propanamin 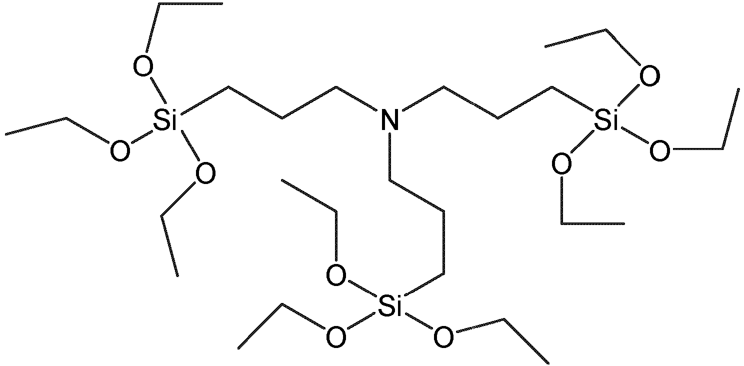
- N1,N1-Bis[3-(trimethoxysilyl)propyl]-1,2-ethanediamin, 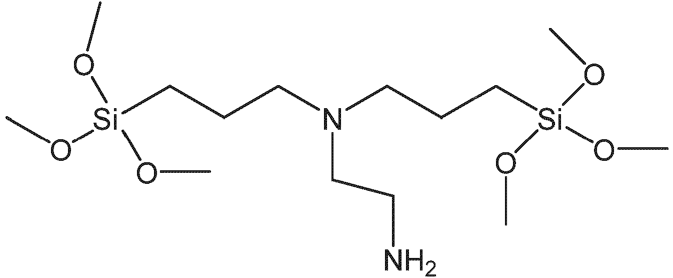
- N1,N1-Bis[3-(triethoxysilyl)propyl]-1,2-ethanediamin, 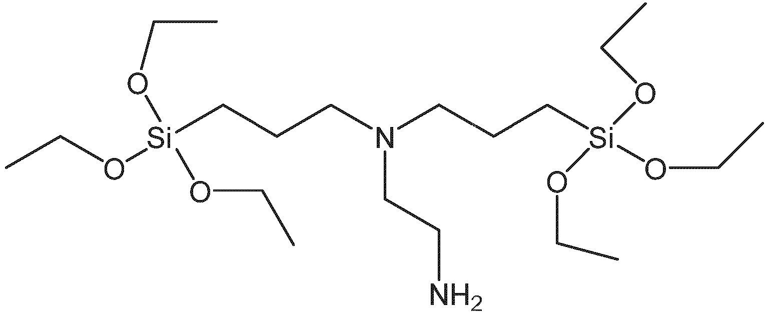
- N,N-Bis[3-(trimethoxysilyl)propyl]-2-propen-1-amin 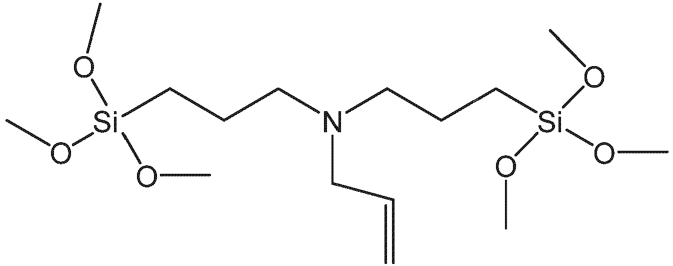
- N,N-Bis[3-(triethoxysilyl)propyl]-2-propen-1-amin

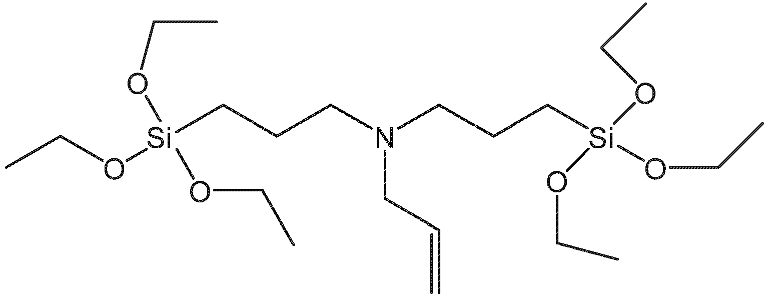

Die vorgenannten organische Siliciumverbindung der Formel (S-II) sind kommerziell erhältlich. Bis(trimethoxysilylpropyl)amine mit der CAS-Nummer 82985-35-1 kann beispielsweise von Sigma-Aldrich käuflich erworben werden.

Bis[3-(triethoxysilyl)propyl]amine mit der CAS-Nummer 13497-18-2 kann zum Beispiel von Sigma-Aldrich käuflich erworben werden.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In einer weiteren bevorzugten Ausführungsform ist ein Verfahren dadurch gekennzeichnet, dass das Mittel (a) ein oder mehrere organische C₁-C₆-Alkoxysilane der Formel (S-II) enthält, die ausgewählt sind aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin,
und/oder deren Kondensationsprodukten.

In Färbeversuchen hat es sich ebenfalls als ganz besonders vorteilhaft herausgestellt, wenn das in dem Verfahren eingesetzte Mittel (a) mindestens ein organisches C₁-C₆-Alkoxysilan der Formel (S-IV) enthält R₉Si(OR₁₀)ₖ(R₁₁)ₘ (S-IV).

Die Verbindungen der Formel (S-IV) sind organische Siliciumverbindungen, die aus Silanen mit einem, zwei oder drei Siliciumatomen ausgewählt sind, wobei die organische Siliciumverbindung eine oder mehrere hydrolysierbare Gruppen pro Molekül umfasst.

Das bzw. die organischen Siliciumverbindungen der Formel (S-IV) können auch als Silane vom Typ der Alkyl-C₁-C₆-Alkoxysilane bezeichnet werden,

R₉Si(OR₁₀)ₖ(R₁₁)ₘ (S-IV),

wobei
- R₉ für eine C₁-C₁₂-Alkylgruppe steht,
- R₁₀ für eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren Ausführungsform ist ein Verfahren dadurch gekennzeichnet, dass das Mittel (a) ein oder mehrere organische C₁-C₆-Alkoxysilane der Formel (S-IV) enthält,

R₉Si(OR₁₀)ₖ(R₁₁)ₘ (S-IV),

wobei
- R₉ für eine C₁-C₁₂-Alkylgruppe steht,
- R₁₀ für eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht,
und/oder deren Kondensationsprodukte.

In den organischen C₁-C₆-Alkoxysilanen der Formel (S-IV) steht der Rest R₉ für eine C₁-C₁₂-Alkylgruppe. Diese C₁-C₁₂-Alkylgruppe ist gesättigt und kann linear oder verzweigt sein. Bevorzugt steht R₉ für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgruppe oder eine n-Octylgruppe.

In den Alkoxysilanen der Formel (S-IV) steht der Rest R₁₀ für eine C₁-C₆-Alkylgruppe. Besonders bevorzugt steht R₁₀ für eine Methylgruppe oder für eine Ethylgruppe.

In den Alkoxysilanen der Formel (S-IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Färbungen mit den besten Waschechtheiten konnten erhalten werden, wenn das kosmetische Mittel mindestens ein organisches C₁-C₆-Alkoxysilan der Formel (S-IV) enthält, bei welchem der Rest k für die Zahl 3 steht. In diesem Fall steht der Rest m für die Zahl 0.

Besonders gut geeignete organische Siliciumverbindungen der Formel (S-IV) sind
- Methyltrimethoxysilan 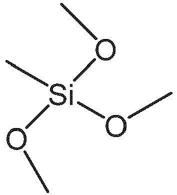
- Methyltriethoxysilan 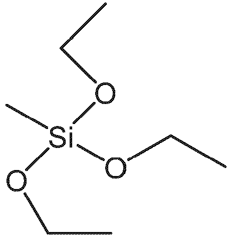
- Ethyltrimethoxysilan 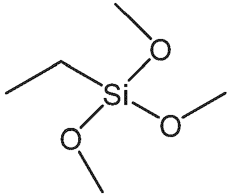
- Ethyltriethoxysilan 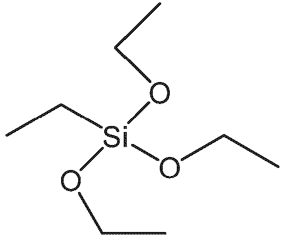
- n-Propyltrimethoxysilan (auch bezeichnet ans Propyltrimethoxysilan) 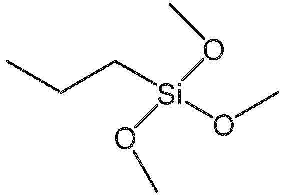
- n-Propyltriethoxysilan (auch bezeichnet als Propyltriethoxysilan) 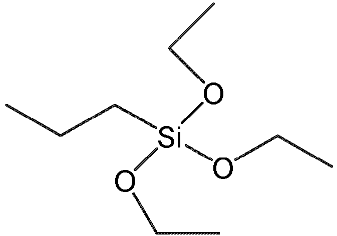
- n-Hexyltrimethoxysilan (auch bezeichnet als Hexyltrimethoxysilan) 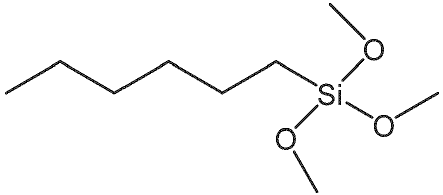
- n-Hexyltriethoxysilan (auch bezeichnet als Hexyltriethoxysilan) 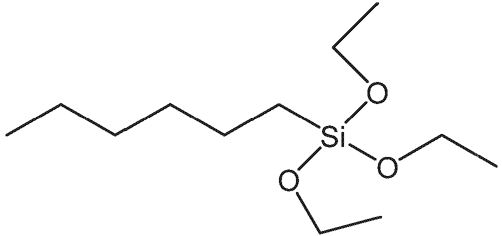
- n-Octyltrimethoxysilan (auch bezeichnet als Octyltrimethoxysilan) 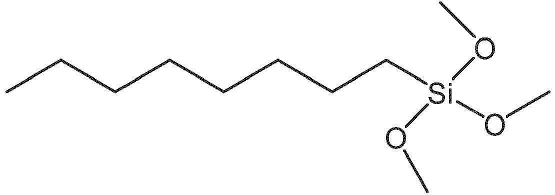
- n-Octyltriethoxysilan (auch bezeichnet als Octyltriethoxysilan) 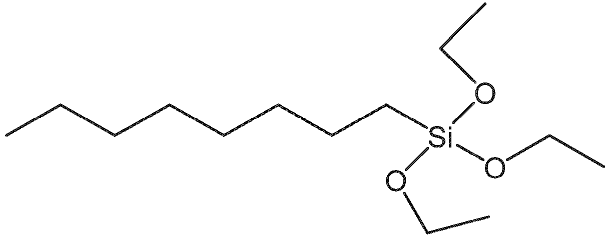
- n-Dodecyltrimethoxysilan (auch bezeichnet als Dodecyltrimethoxysilan) und/oder 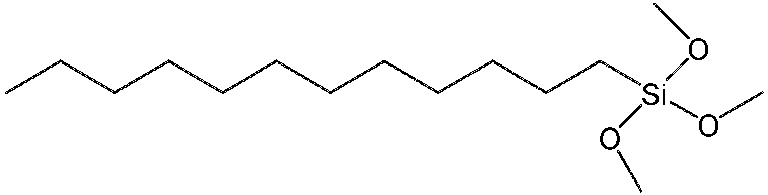
- n-Dodecyltriethoxysilan (auch bezeichnet als Dodecyltriethoxysilan) 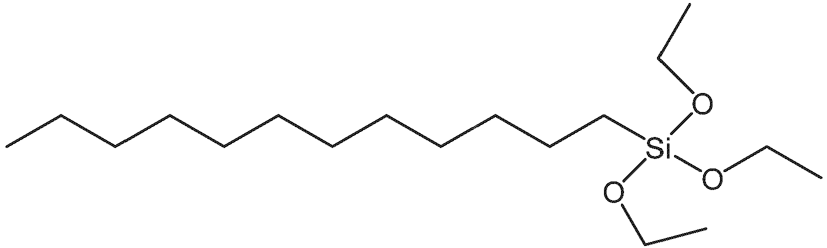
sowie Octadecyltrimethoxysilan und/oder Octadecyltriethoxysilan.

In einer weiteren bevorzugten Ausführungsform ist ein Verfahren dadurch gekennzeichnet, dass das Mittel (a) mindestens ein organisches C₁-C₆-Alkoxysilan der Formel (S-IV) enthält, das ausgewählt ist aus der Gruppe aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
- Octadecyltrimethoxysilan,
- Octadecyltriethoxysilan,
- deren Mischungen
und/oder deren Kondensationsprodukten.

Es hat sich herausgestellt, dass es im Hinblick auf Färbungen von keratinischem Material besonders bevorzugt ist, wenn das kasmetische Mittel zwei strukturell voneinander verschiedene Alkoxysilane enthält.

In einer bevorzugten Ausführungsform ist ein Verfahren dadurch gekennzeichnet, dass das Mittel (a) mindestens ein Alkoxysilan der Formel (S-I) und mindestens ein Alkoxysilan der Formel (S-IV) enthält.

Bei den entsprechenden Hydrolyse bzw. Kondensationsprodukten handelt es sich beispielsweise um die folgenden Verbindungen:

Hydrolyse von C₁-C₆-Alkoxysilan der Formel (S-I) mit Wasser (Reaktionsschema am Beispiel von 3-Aminopropyltriethoxysilan):

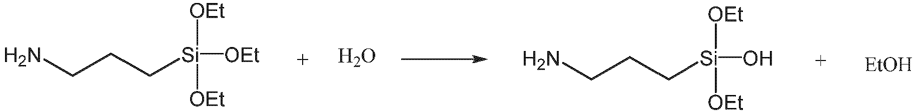

In Abhängigkeit von der eingesetzten Menge an Wasser kann die Hydrolyse-Reaktion auch mehrfach pro eingesetztem C₁-C₆-Alkoxysilan stattfinden: 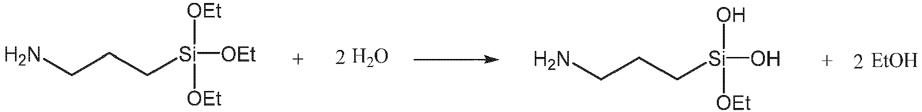 bzw.

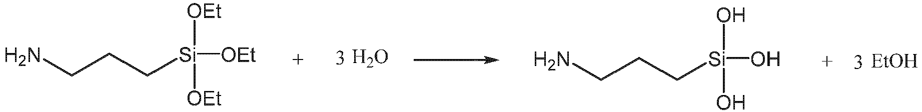

Hydrolyse von C₁-C₆-Alkoxysilan der Formel (S-IV) mit Wasser (Reaktionsschema am Beispiel von Methyltrimethoxysilan):

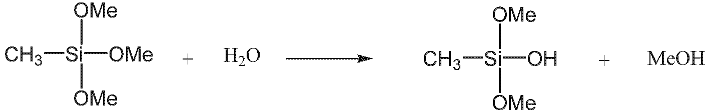

In Abhängigkeit von der eingesetzten Menge an Wasser kann die Hydrolyse-Reaktion auch mehrfach pro eingesetztem C₁-C₆-Alkoxysilan stattfinden: 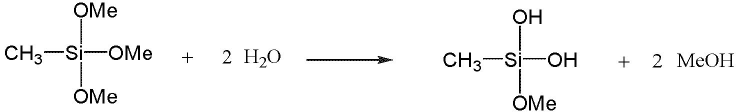 bzw.

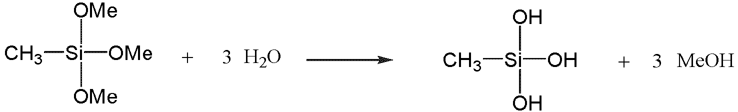

Mögliche Kondensationsreaktionen sind beispielsweise (gezeigt anhand des Gemisches (3-Aminopropyl)triethoxysilan und Methyltrimethoxysilan): 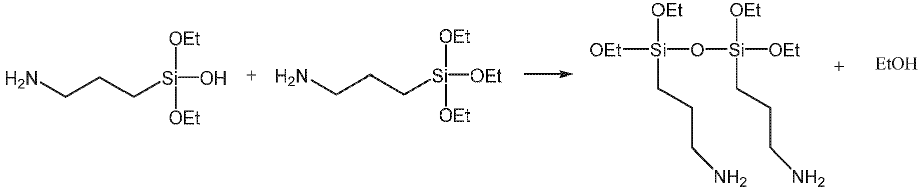 und/oder 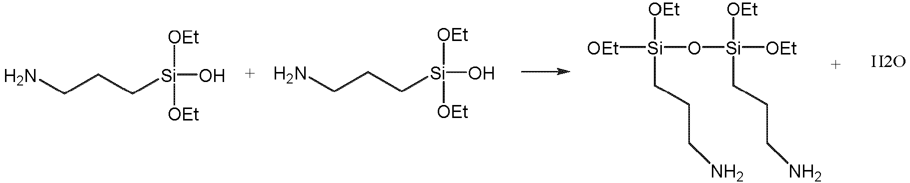 und/oder 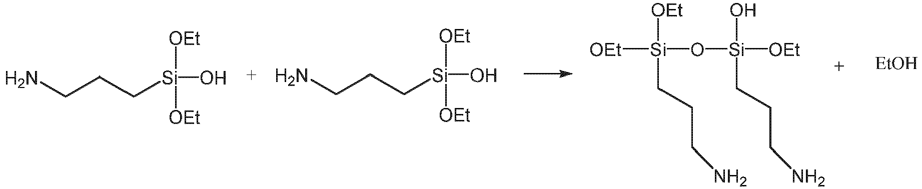 und/oder 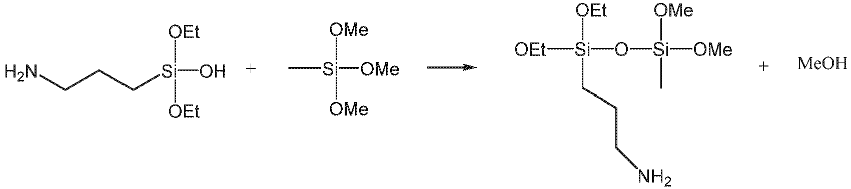 und/oder 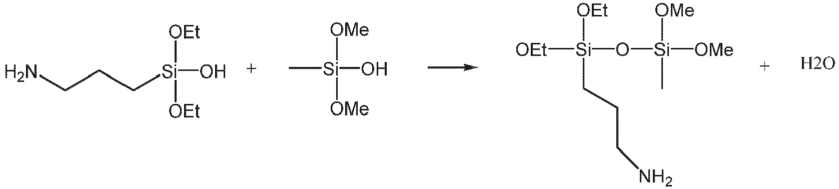 und/oder 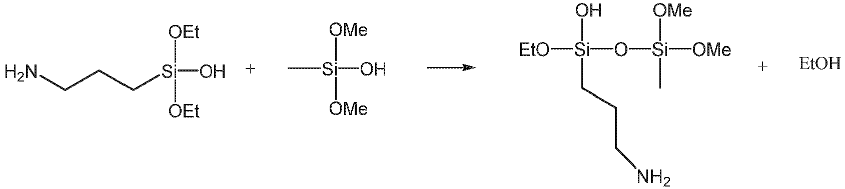 und/oder

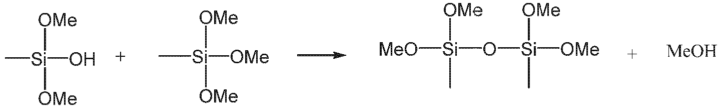

In den obigen beispielhaften Reaktionsschemata ist jeweils die Kondensation zu einem Dimer gezeigt, jedoch sind auch weitergehende Kondensationen zu Oligomeren mit mehreren Silan-Atomen möglich und auch bevorzugt.

An diesen Kondensationsreaktionen können sowohl partiell hydrolysierte als auch vollständig hydrolysierte C₁-C₆-Alkoxysilane der Formel (S-I) teilnehmen, die eine Kondensation mit noch nicht abreagierten, partiell oder auch vollständig hydrolysierten C₁-C₆-Alkoxysilanen der Formel (S-I) durchlaufen. In diesem Fall reagieren die C₁-C₆-Alkoxysilane der Formel (S-I) mit sich selbst.

Weiterhin können an den Kondensationsreaktionen auch sowohl partiell hydrolysierte als auch vollständig hydrolysierte C₁-C₆-Alkoxysilane der Formel (S-I) teilnehmen, die eine Kondensation mit noch nicht abreagierten, partiell oder auch vollständig hydrolysierten C₁-C₆-Alkoxysilanen der Formel (S-IV) durchlaufen. In diesem Fall reagieren die C₁-C₆-Alkoxysilane der Formel (S-I) mit den C₁-C₆-Alkoxysilanen der Formel (S-IV).

Weiterhin können an den Kondensationsreaktionen auch sowohl partiell hydrolysierte als auch vollständig hydrolysierte C₁-C₆-Alkoxysilane der Formel (S-IV) teilnehmen, die eine Kondensation mit noch nicht abreagierten, partiell oder auch vollständig hydrolysierten C₁-C₆-Alkoxysilanen der Formel (S-IV) durchlaufen. In diesem Fall reagieren die C₁-C₆-Alkoxysilane der Formel (S-IV) mit sich selbst.

Das Mittel (a) kann ein oder mehrere organische C₁-C₆-Alkoxysilane in verschiedenen Mengenanteilen enthalten. Diese bestimmt der Fachmann in Abhängigkeit von der gewünschten Anwendung. Im Fall von Färbungen von keratinischem Material kann die Menge beispielsweise von der Dicke des Silan-Coatings auf dem keratinischen Material und von der Menge des zu behandelnden keratinischen Material abhängen.

Besonders lagerstabile Mittel (a) mit sehr gutem Färberesultat bei der Anwendung auf keratinischem Material konnten erhalten werden, wenn das kosmetische Mittel - bezogen auf sein Gesamtgewicht - ein oder mehrere organische C₁-C₆-Alkoxysilane und/oder die Kondensationsprodukte hiervon in einer Gesamtmenge von 30 bis 85 Gew.-%, bevorzugt von 35 bis 80 Gew.-%, weiter bevorzugt von 40 bis 75 Gew.-%, noch weiter bevorzugt von 45 bis 70 Gew.-% und ganz besonders bevorzugt von 50 bis 65 Gew.-% enthält.

Es kann bevorzugt sein, dass das anwendungsbereite Mittel (a) neben der erfindungsgemäßen Pigmentsuspension und dem/den organischen C₁-C₆-Alkoxysilan(en) weitere Inhaltsstoffe enthält.

Das kosmetische Mittel enthält mit den Alkoxysilanen eine Klasse von hochreaktiven Verbindungen, die bei ihrer Anwendung eine Hydrolyse oder Oligomerisierung und/oder Polymerisierung eingehen können. Zur Vermeidung der vorzeitigen Oligomerisierung bzw. Polymerisierung kann es für den Anwender von wesentlichem Vorteil sein, das anwendungsbereite kosmetische Mittel erst kurz vor der Anwendung herzustellen.

Zur Vermeidung der vorzeitigen Oligomerisierung bzw. Polymerisierung ist es für den Anwender von wesentlichem Vorteil, das anwendungsbereite Mittel (a) erst kurz vor der Anwendung herstellen.

So kann der Anwender beispielsweise ein Mittel (a'), welches die Alkoxysilane (a1) enthält, mit einem Mittel (a"), umfassend die erfindungsgemäße Pigmentsuspension, verrühren oder verschütteln. Diese Mischung aus (a') und (a") kann der Anwender entweder direkt nach ihrer Herstellung oder nach einer kurzen Reaktionszeit von 10 Sekunden bis 20 Minuten - auf die keratinischen Materialien applizieren. Im Anschluss daran kann der Anwender das Mittel (b) anwenden.

Das Verfahren Färben von keratinischen Material umfasst neben der Anwendung des Mittels (a) auch die Anwendung des Mittels (b). Das Mittel (b) ist dadurch gekennzeichnet, dass es mindestens ein Versiegelungsreagenz (b1) enthält.

Das Mittel (b) ist ein Nachbehandlungsmittel und die Anwendung des Mittels (b) auf das mit Mittel (a) behandelte keratinische Material führt dazu, dass die in dem Verfahren erzielten Färbungen haltbarer gemacht werden. Insbesondere kann durch Anwendung des Mittels (b) die Waschechtheit und die Reibechtheit der im Verfahren erhaltenen Färbungen verbessert werden.

Es ist bevorzugt, dass das Versiegelungsreagenz (b1) eine Verbindung ausgewählt aus der Gruppe bestehend aus filmbildenden Polymeren, Alkalisierungsmitteln, Acidifizierungsmittel und Mischungen daraus umfasst.

Im Fall, dass das Mittel (b) die erfindungsgemäße Pigmentsuspension enthält, kann es ebenfalls bevorzugt sein, das anwendungsbereite Mittel (b) durch Vermischen von zwei Mitteln (b') und (a") herzustellen. Das Mittel (b') enthält in dieser Ausführungsform das Versiegelungsreagenz (b1) und das Mittel (a") enthält die Pigmentsuspension.

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Ein dritter Gegenstand der vorliegenden Erfindung ist daher eine Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') enthält:
   (a1) mindestens ein oder mehrerer organische C₁-C₆-Alkoxysilane,
- einen zweiten Container mit einem Mittel (a"), wobei das Mittel (a") enthält:
   (a2) eine erfindungsgemäße Pigmentsuspension, und
- einen dritten Container mit einem Mittel (b'), wobei das Mittel (b') enthält:
   (b1) ein Versiegelungsreagenz.

Bezüglich weiterer bevorzugter Ausführungsformen der Mehrkomponenten-Verpackungseinheit (Kit-of-parts) gilt mutatis mutandis das zu den Pigmentsuspensionen und/oder Verfahren Gesagte.

### Beispiel 1

Es wurden die folgenden Formulierungen hergestellt (sofern nichts anderes angegeben ist, sind alle Angaben in Gew.-% Aktivsubstanz)

### Mittel (a)

| Mittel (a) | Gew.-% |
|---|---|
| (3-Aminopropyl)triethoxysilan | 20 |
| Methyltriethoxysilan | 70 |
| Wasser | ad 100 |
| | |

| Mittel (b') | Gew.-% |
|---|---|
| Ethylene/Sodium Acrylate Copolymer (b1) (25%ige Lösung) | 15 |
| Wasser | ad 100 |

| Pigmentsuspension | Gew.-% |
|---|---|
| Timiron SynWhite Satin (ex Merck) | 45 |
| 1,2-Propanol | 12 |
| 2-Propanol | 30 |
| Hydroxypropylmethylcellulose | 1 |
| Wasser | ad 100 |

Das anwendungsbereite Mittel (b) wurde durch Vermischen von 60 g des Mittels (b') und 40 g der Pigmentsuspension hergestellt.

Das Mittel (a) wurde jeweils in eine Haarsträhne (Kerling, Euronaturhaar weiß) einmassiert, und für 1 Minute einwirken gelassen. Danach wurde das Mittel (a) mit Wasser ausgespült.

Im Anschluss daran wurde das Mittel (b) auf die Haarsträhne appliziert, für 5 Minute einwirken gelassen und danach ebenfalls mit Wasser ausgespült.

## Patentansprüche

1. Pigmentsuspension, umfassend
a) mindestens eine farbgebende Verbindung aus der Gruppe der Pigmente, wobei das Pigment ein Substratplättchen aus natürlichem Glimmer oder synthetischem Glimmer umfasst,
b) mindestens einen C₁-C₁₀-Alkohol,
c) mindestens ein Diol, ausgewählt aus der Gruppe bestehend aus Ethylenglycol, 1,2-Propylenglycol, 1,3-Propylenglycol und Mischungen daraus,
d) mindestens ein Verdickungsmittel und
e) Wasser.

2. Pigmentsuspension gemäß Anspruch 1, **dadurch gekennzeichnet, dass** das Pigment ein Substratplättchen aus synthetischem Glimmer (INCI: Synthetic Fluorphlogopite) umfasst.

3. Pigmentsuspension gemäß Anspruch 1 oder 2, **dadurch gekennzeichnet, dass** der mindestens eine C₁-C₁₀-Alkohol ausgewählt aus der Gruppe bestehend aus Ethanol, 2-Propanol und Mischungen davon.

4. Pigmentsuspension gemäß einem der Ansprüche 1 bis 3, **dadurch gekennzeichnet, dass** das mindestens eine Verdickungsmittel ausgewählt aus der Gruppe bestehend aus Propylcellulose, Methylethylcellulose, Carboxymethylcellulose, Hydroxyethylcellulose, Methylhydroxyethylcellulose, Ethylhydroxyethylcellulose, Hydroxypropylcellulose, Hydroxypropylmethylcellulose, Carboxymethylhydroxyethylcellulose, Sulfoethylcellulose, Carboxymethylsulfoethylcellulose, Hydroxypropylsulfoethylcellulose, Hydroxyethylsulfoethylcellulose, Methylethylhydroxyethylcellulose, Methlylsulfoethylcellulose, Ethylsulfoethylcellulose und Mischungen daraus enthält.

5. Verfahren zum Färben von keratinischem Material, insbesondere menschlichen Haaren, umfassend die folgenden Schritte:
- Anwendung eines Mittels (a) auf dem keratinischem Material, wobei das Mittel (a) enthält:
(a1) mindestens ein organisches C₁-C₆-Alkoxysilan, und
- Anwendung eines Mittels (b) auf dem keratinischen Material, wobei das Mittel (b) enthält:
(b1) mindestens ein Versiegelungsreagenz,
wobei mindestens eines der Mittel (a) und (b) ferner eine Pigmentsuspension nach einem der Ansprüche 1 bis 4 umfasst.

6. Verfahren zum Färben von keratinischem Material gemäß Anspruch 5, **dadurch gekennzeichnet, dass** das Mittel (a) ein oder mehrerer organische C₁-C₆-Alkoxysilane der Formel (S-I) und/oder (S-II) Und/oder (S-IV) enthält,
R₁R₂N-L-Si(OR₃)ₐ(R₄)_{b} (S-I)
wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃, R₄ unabhängig voneinander für eine C₁-C₆-Alkylgruppe stehen,
- a, für eine ganze Zahl von 1 bis 3 steht, und
- b für die ganze Zahl 3 - a steht, und
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (S-II),
wobei
- R5, R5', R5", R6, R6' und R6" unabhängig voneinander für eine C₁-C₆-Alkylgruppe stehen,
- A, A', A", A‴ und A"" unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (S-III) stehen,
- (Aʺʺ)-Si(R₆'')_{d}''(OR₅'')_{c}'' (S-III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist,
und/oder
R₉Si(OR₁₀)ₖ(R₁₁)ₘ (S-IV),
wobei
- R₉ für eine C₁-C₁₂-Alkylgruppe steht,
- R₁₀ für eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

7. Verfahren zum Färben von keratinischem Material gemäß Anspruch 5 oder 6, **dadurch gekennzeichnet, dass** das Mittel (a) mindestens zwei strukturell voneinander verschiedene organische C₁-C₆-Alkoxysilane enthält.

8. Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') enthält:
(a1) mindestens ein oder mehrerer organische C₁-C₆-Alkoxysilane,
- einen zweiten Container mit einem Mittel (a"), wobei das Mittel (a") enthält:
(a2) eine Pigmentsuspension gemäß einem der Ansprüche 1 bis 4, und
- einen dritten Container mit einem Mittel (b"), wobei das Mittel (b") enthält:
(b1) ein Versiegelungsreagenz.

## Claims

1. Pigment suspension comprising
a) at least one coloring compound from the group of pigments, wherein the pigment comprises a substrate platelet of natural mica or synthetic mica,
b) at least one C₁-C₁₀ alcohol,
c) at least one diol selected from the group consisting of ethylene glycol, 1,2-propylene glycol, 1,3-propylene glycol and mixtures thereof,
d) at least one thickening agent, and
e) water.

2. Pigment suspension according to claim 1, **characterized in that** the pigment comprises a substrate platelet of synthetic mica (INCI: Synthetic Fluorphlogopite).

3. Pigment suspension according to claim 1 or 2, **characterized in that** the at least one C₁-C₁₀ alcohol is selected from the group consisting of ethanol, 2-propanol, and mixtures thereof.

4. Pigment suspension according to any one of claims 1 to 3, **characterized in that** the at least one thickening agent is selected from the group consisting of propyl cellulose, methyl ethyl cellulose, carboxymethyl cellulose, hydroxyethyl cellulose, methyl hydroxyethyl cellulose, ethyl hydroxyethyl cellulose, hydroxypropyl cellulose, hydroxypropyl methylcellulose, carboxymethyl hydroxyethylcellulose, sulfoethylcellulose, carboxymethyl sulfoethylcellulose, hydroxypropyl sulfoethylcellulose, hydroxyethyl sulfoethylcellulose, methylethyl hydroxyethylcellulose, methyl sulfoethylcellulose, ethyl sulfoethylcellulose and mixtures thereof.

5. A method for coloring keratinous material, in particular human hair, comprising the following steps:
- Applying an agent (a) to the keratinous material, wherein the agent (a) contains:
(a1) at least one organic C₁-C₆ alkoxysilane, and
- applying an agent (b) to the keratinous material, wherein the agent (b) comprises:
(b1) at least one sealing reagent,
wherein at least one of the agents (a) and (b) further comprises a pigment suspension according to any one of claims 1 to 4.

6. A method for coloring keratinous material according to claim 4, **characterized in that** agent (a) contains one or more organic C₁-C₆ alkoxysilanes of formula (S-I) and/or (S-II) and/or (S-IV),
R₁R₂N-L-Si(OR₃)ₐ(R₄)_{b} (S-I)
where
- R₁, R₂ independently represent a hydrogen atom or a C₁-C₆ alkyl group,
- L represents a linear or branched, divalent C₁-C₂₀ alkylene group,
- R₃, R₄ independently represent a C₁-C₆ alkyl group,
- a represents an integer from 1 to 3, and
- b represents the integer 3 - a, and
(R₅₀)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (S-II),
wherein
- R5, R5', R5", R6, R6' and R6'' independently represent a C₁-C₆ alkyl group,
- A, A', A", A‴ and Aʺʺ independently represent a linear or branched, divalent C₁-C₂₀ alkylene group,
- R₇ and R₈ independently represent a hydrogen atom, a C₁-C₆ alkyl group, a hydroxy-C₁-C₆ alkyl group, a C₂-C₆ alkenyl group, an amino-C₁-C₆ alkyl group or a group of formula (S-III),
- (Aʺʺ)-Si(R₆'')_{d}''(OR₅'')_{c}'' (S-III),
- c is an integer from 1 to 3,
- d is the integer 3 - c,
- c' is an integer from 1 to 3,
- d' is the integer 3 - c',
- c'' is an integer from 1 to 3,
- d'' is the integer 3 - c",
- e is 0 or 1,
- f is 0 or 1,
- g is 0 or 1,
- h stands for 0 or 1,
- with the proviso that at least one of the radicals e, f, g and h is different from 0,
and/or
R₉Si(OR₁₀)ₖ(R₁₁)ₘ (S-IV),
where
- R₉ represents a C₁-C₁₂ alkyl group,
- R₁₀ represents a C₁-C₆ alkyl group,
- R₁₁ represents a C₁-C₆ alkyl group
- k represents an integer from 1 to 3, and
- m represents the integer 3 - k.

7. A method for coloring keratinous material according to claim 5 or 6, **characterized in that** the agent (a) contains at least two structurally different organic C₁-C₆ alkoxysilanes.

8. Multi-component packaging unit (kit of parts) for dyeing keratinous material, comprising, packaged separately from each other
- a first container with an agent (a'), wherein the agent (a') contains:
(a1) at least one or more organic C₁-C₆ alkoxysilanes,
- a second container with an agent (a"), wherein the agent (a") contains:
(a2) a pigment suspension according to one of claims 1 to 4, and
- a third container with an agent (b"), wherein the agent (b") contains:
(b1) a sealing reagent.

## Revendications

1. Suspension pigmentaire comprenant
a) au moins un composé colorant du groupe des pigments, le pigment comprenant une plaquette de substrat en mica naturel ou synthétique,
b) au moins un alcool en C₁ à C₁₀,
c) au moins un diol choisi dans le groupe constitué par l'éthylène glycol, le 1,2-propylène glycol, le 1,3-propylène glycol et leurs mélanges,
d) au moins un agent épaississant et
e) de l'eau.

2. Suspension pigmentaire selon la revendication 1, **caractérisée en ce que** le pigment comprend une plaquette de substrat en mica synthétique (INCI : Synthetic Fluorphlogopite).

3. Suspension pigmentaire selon la revendication 1 ou 2, **caractérisée en ce que** le au moins un alcool en C₁ à C₁₀ est choisi dans le groupe constitué par l'éthanol, le 2-propanol et leurs mélanges.

4. Suspension pigmentaire selon l'une des revendications 1 à 3, **caractérisée en ce que** le au moins un agent épaississant est choisi dans le groupe constitué par la propylcellulose, la méthyléthylcellulose, la carboxyméthylcellulose, l'hydroxyéthylcellulose, la méthylhydroxyéthylcellulose, l'éthylhydroxyéthylcellulose, l'hydroxypropylcellulose, hydroxypropylméthylcellulose, carboxyméthylhydroxyéthylcellulose, sulfoéthylcellulose, carboxyméthylsulfoéthylcellulose, hydroxypropylsulfoéthylcellulose, hydroxyéthylsulfoéthylcellulose, méthyléthylhydroxyéthylcellulose, méthylsulfoéthylcellulose, éthylsulfoéthylcellulose et leurs mélanges.

5. Procédé de coloration d'une matière kératinique, en particulier des cheveux humains, comprenant les étapes suivantes :
- application d'un agent (a) sur la matière kératinique, l'agent (a) contenant :
(a1) au moins un alcoxysilane organique en C₁ à C₆, et
- application d'un agent (b) sur la matière kératinique, l'agent (b) contenant :
(b1) au moins un réactif de scellement,
au moins l'un des agents (a) et (b) comprenant en outre une suspension de pigment selon l'une des revendications 1 à 4.

6. Procédé de coloration d'une matière kératinique selon la revendication 4, **caractérisé en ce que** l'agent (a) contient un ou plusieurs alcoxysilanes organiques en C₁ à C₆ de formule (S-I) et/ou (S-II) et/ou (S-IV),
R₁R₂N-L-Si(OR₃)ₐ(R₄)_{b} (S-I)
où
- R₁, R₂ représentent indépendamment l'un de l'autre un atome d'hydrogène ou un groupe alkyle en C₁-C₆,
- L représente un groupe alkylène en C₁-C₂₀ divalent, linéaire ou ramifié,
- R₃, R₄ représentent indépendamment l'un de l'autre un groupe alkyle en C₁-C₆,
- a représente un nombre entier de 1 à 3, et
- b représente le nombre entier 3 - a, et
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (S-II),
où
- R5, R5', R5‴, R6, R6" et R6‴ représentent indépendamment les uns des autres un groupe alkyle en C₁-C₆,
- A, A", A‴, A"" et A‴‴ représentent indépendamment les uns des autres un groupe alkylène en C₁-C₂₀ divalent, linéaire ou ramifié,
- R₇ et R₈ représentent indépendamment l'un de l'autre un atome d'hydrogène, un groupe alkyle en C₁-C₆, un groupe hydroxyalkyle en C₁-C₆, un groupe alcényle en C₂-C₆, un groupe aminoalkyle en C₁-C₆ ou un groupement de formule (S-III), -
(A‴‴)-Si(R₆ʺʺ)_{d}ʺʺ(OR₅ʺʺ)_{c}ʺʺ (S-III),
- c représente un nombre entier compris entre 1 et 3,
- d représente le nombre entier 3 - c,
- c" représente un nombre entier compris entre 1 et 3,
- d' représente le nombre entier 3 - c',
- c" représente un nombre entier compris entre 1 et 3,
- d" représente le nombre entier 3 - c",
- e représente 0 ou 1,
- f représente 0 ou 1,
- g représente 0 ou 1,
- h représente 0 ou 1,
- à condition qu'au moins l'un des radicaux e, f, g et h soit différent de 0,
et/ou
R₉Si(OR₁₀)ₖ(R₁₁)ₘ (S-IV),
où
- R₉ représente un groupe alkyle en C₁ à C₁₂,
- R₁₀ représente un groupe alkyle en C₁ à C₆,
- R₁₁ représente un groupe alkyle en C₁ à C₆
- k représente un nombre entier de 1 à 3, et
- m représente le nombre entier 3 - k.

7. Procédé de coloration de matière kératinique selon la revendication 5 ou 6, **caractérisé en ce que** l'agent (a) contient au moins deux alcoxysilanes organiques en C₁ à C₆ structurellement différents les uns des autres.

8. Ensemble d'emballage à plusieurs composants (kit de pièces) pour la coloration de matière kératinique, comprenant, conditionnés séparément les uns des autres
- un premier récipient contenant un agent (a'), l'agent (a') contenant :
(a1) au moins un ou plusieurs alcoxysilanes organiques en C₁ à C₆,
- un deuxième récipient contenant un agent (a‴), l'agent (a‴) contenant :
(a2) une suspension pigmentaire selon l'une des revendications 1 à 4, et
- un troisième récipient contenant un agent (b‴), l'agent (b"') contenant :
(b1) un réactif de scellement.
